Europäisches Patentamt

(19) European Patent Office

Office européen des brevets

(11) **EP 0 521 963 B1**

(12) **EUROPEAN PATENT SPECIFICATION**

(45) Date of publication and mention
of the grant of the patent:
**12.08.1998 Bulletin 1998/33**

(21) Application number: **91906643.1**

(22) Date of filing: **21.03.1991**

(51) Int Cl.[6]: **C07K 14/575**, A61K 38/22

(86) International application number:
**PCT/US91/01896**

(87) International publication number:
**WO 91/14446 (03.10.1991 Gazette 1991/23)**

(54) **CRF ANALOGS**

CRF-ANALOGE

ANALOGUES DE CRF

(84) Designated Contracting States:
**AT BE CH DE DK ES FR GB GR IT LI LU NL SE**

(30) Priority: **23.03.1990 US 498230**

(43) Date of publication of application:
**13.01.1993 Bulletin 1993/02**

(73) Proprietor: **THE SALK INSTITUTE FOR
BIOLOGICAL STUDIES
La Jolla California 92037 (US)**

(72) Inventors:
• **RIVIER, Jean, Edouard, Frederic
La Jolla, CA 92037 (US)**
• **VALE, Wylie, Walker, Jr.
La Jolla, CA 92037 (US)**

(74) Representative: **Lawrence, Malcolm Graham et al
Hepworth, Lawrence, Bryer & Bizley
Merlin House
Falconry Court
Baker's Lane
Epping Essex CM16 5DQ (GB)**

(56) References cited:
EP-A- 0 153 845          EP-A- 0 361 954
WO-A-89/12646          WO-A-90/03392
US-A- 3 761 459

• **PEPTIDE CHEMISTRY 1988, OSAKA pages 597 -
600 J.RIVIER 'CRF: CHARACTERIZATION OF
NEW ANALOGS'**

• **B.WEINSTEIN 'CHEMISTRY AND
BIOCHEMISTRY OF AMINO ACIDS, PEPTIDES
AND PROTEINS, VOLUME 7' 1983 , MARCEL
DEKKER,INC. , NEW YORK CHAPTER 5;
A.F.SPATOLA, 'PEPTIDE BACKBONE
MODIFICATIONS'**

• **CHEMICAL ABSTRACTS, Vol. 109, No. 19,
issued 1988, (Columbus, Ohio, US), RIVIER, J.,
"Corticotropin releasing factor. Characterization
of new analogs", see page 768, col. 1, Abstract
No. 170909q. Pept. Chem. 1987, pp. 597-600.**

• **Science, Vol. 224, issued 25 May 1984, RIVIER et
al, "Synthetic competitive antagonists of
Corticotropin-Releasing Factor: Effect on ACTH
secretion in the rat", pp. 889-891. See page 890,
col. 3, par. 2.**

• **Proceedings of the National Academy of Science
USA, Vol. 80, issued August 1983, RIVIER et al,
"Characterization of rat hypothalamic
corticotropin-releasing factor", pp. 4851-4855,
see Abstract, col. 1.**

• **The EMBO Journal, vol. 2, No. 5, issued 1983,
SHIBAHARA et al, "Isolation and sequence
analysis of the human corticotropin-releasing
precursor gene", pp. 775-779, see p. 778, col. 1,
lines 1-7.**

• **CHEMICAL ABSTRACTS, Vol. 96, issued 1982,
(Columbus, Ohio, US), TOMA et al,
"Conformational analysis of corticotropin
(ACTH) and conformation-activity relationship",
see page 105, col. 2, Abstract No. 97839y,
Biochemie 1981, Vol. 63 (11-12), pp 907-10.**

- **CHEMICAL ABSTRACTS, Vol. 98, issued 1983, (Columbus, Ohio, US), LOW et al, "Biological and conformational properties of some corticotropin analogs containing D-amino acids", see page 67, col. 2, Abstract No. 65649M, Pept. Proc. Eur. Pept. Symp. 16th, 1980, pp. 513-19.**

**Description**

This invention is directed to peptides and to methods for pharmaceutical treatment of mammals using such peptides. More specifically, the invention relates to analogs of the hentetracontapeptide CRF, to pharmaceutical compositions containing such CRF analogs and to methods of treatment of mammals using such CRF analogs.

Experimental and clinical observations have supported the concept that the hypothalamus plays a key role in the regulation of adenohypophysial corticotropic cells secretory functions. Over 25 years ago, Guillemin, Rosenberg and Saffran and Schally independently demonstrated the presence of factors in hypothalamus which would increase the rate of ACTH secretion by the pituitary gland incubated in vitro or maintained in an organ culture. None of the secretagogs characterised met the criteria expected of the physiologic corticotropin releasing factor (CRF) until ovine CRF (oCRF) was characterized in 1981 and, as disclosed in U.S. Patent No. 4,415,558, was found to have the formula: H-Ser-Gln-Glu-Pro-Pro-Ile-Ser-Leu-Asp-Leu-Thr-Phe-His-Leu-Leu-Arg-Glu-Val-Leu-Glu-Met-Thr-Lys-Ala-Asp-Gln-Leu-Ala-Gln-Gln-Ala-His-Ser-Asn-Arg-Lys-Leu-Leu-Asp-Ile-Ala-$NH_2$, oCRF lowers blood pressure in mammals and stimulates the secretion of ACTH and β-endorphin.

Rat CRF (rCRF) has been isolated, purified and characterized as a hentetracontapeptide having the formula: H-Ser-Glu-Glu-Pro-Pro-Ile-Ser-Leu-Asp-Leu-Thr-Phe-His-Leu-Leu-Arg-Glu-Val-Leu-Glu-Met-Ala-Arg-Ala-Glu-Gln-Leu-Ala-Gln-Gln-Ala-His-Ser-Asn-Arg-Lys-Leu-Met-Glu-Ile-Ile-$NH_2$. It may alternatively be referred to as rat amunine. The formula of human CRF has now been determined to be the same as that of rCRF, and the terms rCRF and hCRF are used interchangeably. Synthetic rCRF and oCRF stimulate ACTH and β-endorphin-like activities (β-END-LI) in vitro and in vivo and substantially lower blood pressure. EP-A-0153845 discloses CRF analogs that contain D-Pro as the fourth amino acid residue and CML as the thirty seventh residue. Peptide Chemistry 1988, p.597-600 discloses that the introduction of D-Phe 12 to compounds belonging to the same relevant field as the invention described herein restores a high level of activity.

Analogs of these 41 residue CRF peptides have been discovered which exhibit at least about the same biological activity in vitro as the native peptides and have substantially longer duration of biological effect in vivo. In these peptides the residue in the 37 position is substituted with a methyl group on its α-carbon atom and they may have at least one and preferably at least 2 of the following D-isomer substitutions: D-Phe in the 12-position, D-Glu in the 20-position, D-Ala in the 24 position and D-His in the 32 position. Norleucine may be substituted in the 18, 21 and/or 30 positions. The residues in the 8, 12, 19, 21, 22, 24, 27, 28, 32, 33, 36, 38, 40 and/or 41 positions may also have a $C^{\alpha}Me$ substitution. The N-terminus can be optionally shortened by from one to a sequence of up to 6 residues, and the N-terminal residue may be acylated. Pharmaceutical compositions in accordance with the invention include such CRF analogs, or nontoxic addition salts thereof, dispersed in a pharmaceutically or veterinarily acceptable liquid or solid carrier. The administration of such peptides or pharmaceutically or veterinarily acceptable addition salts thereof to mammals, particularly humans, in accordance with the invention may be carried out for the regulation of secretion of ACTH, β-endorphin, β-lipotropin, other products of the pro-opiomelanocortin gene and corti-costerone and/or for the lowering of blood pressure and/or for affecting mood, behavioural and gastrointestinal functions and autonomic nervous system activities. Furthermore CRF analogs may be used for the evaluation of the status of pituitary, cardiovascular, gastrointestinal or central nervous system functions.

The nomenclature used to define the peptides is that specified by Schroder & Lubke, "The Peptides", Academic Press (1965) wherein, in accordance with conventional representation, the amino group appears to the left and the carboxyl group to the right. The standard 3-letter abbreviations to identify the alpha-amino acid residues, and where the amino acid residue that is represented unless otherwise expressly indicated, e.g. Ser = L-serine, Orn = ornithine, Nle = norleucine, Nva = norvaline and Har = homoarginine. In addition the following abbreviations are used: leu = either L-leucine or $C^{\alpha}CH_3$-L-leucine (CML); ala = either L-alanine or $C^{\alpha}CH_3$-L-alanine (CMA).

The invention provides analogs of CRF having the following Formula (I): Z-D-Pro-Pro-Ile-Ser-Leu-Asp-Leu-Thr-D-Phe-His-Leu-Leu-Arg-Glu-Val-Leu-$R_{20}$-Nle-$R_{22}$-$R_{23}$-$R_{24}$-$R_{25}$-Gln-Leu-Ala-Gln-Gln-Ala-$R_{32}$-Ser-Asn-Arg-Lys-CML-Nle-$R_{39}$-Ile-$R_{41}$-Y wherein $R_{20}$ is Glu or D-Glu; $R_{22}$ is Ala or Thr; $R_{23}$ is Arg or Lys; $R_{24}$ is Ala or D-Ala; $R_{25}$ is Glu or Asp; $R_{32}$ is His or D-His; $R_{39}$ is Glu or Asp; $R_{41}$ is Ile or Ala; Y is amino, methylamino or ethylamino; and Z is an acyl group of up to seven carbon atoms or is hydrogen.

Nontoxic addition salts of these peptides can be used as well. Preferably either D-Phe in the 12-position or D-Glu in the 20-position is also present. These analogs remain potent even if slightly shortened at the N-terminus, i.e., by a sequence of up to about 6 residues.

Instead of the simple amide at the C-terminus, a lower alkyl substituted amide, e.g., methylamide or ethylamide may be incorporated.

The peptides can be synthesised by a suitable method, such as by exclusively solid-phase techniques, by partial solid-phase techniques, by fragment condensation or by classical solution addition. Common to chemical syntheses of peptides is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with various of these residues having side-chain protecting groups.

Thus, chemical synthesis of such a peptide analog may result in the formation of an intermediate of the Formula (IA): $X^1$-D-Pro-Pro-Ile-Ser($X_2$)-Leu-Asp($X_5$)-Leu-Thr($X_2$)-D-Phe-His($X_7$)-Leu-Leu-Arg($X_3$)-Glu($X^5$)-Val-Leu-$R_{20}$($X^5$)-Nle-$R_{22}$($X^2$)-$R_{23}$($X^3$ or $X^6$)-$R_{24}$-$R_{25}$($X^5$)-Gln($X^4$)-Leu-Ala-Gln($X^4$)-Gln($X^4$)-Ala-$R_{32}$($X^7$)-Ser($X^2$)-Asn($X^4$)-Arg($X^3$)-Lys($X^6$)-CML-Nle-$R_{39}$($X^5$)-Ile-$R_{41}$-($X^8$) or an N-terminally shortened version thereof, wherein: the R-groups are as here-inbefore defined.

$X^1$ is either hydrogen or an alpha-amino protecting group. The alpha-amino protecting groups contemplated by $X^1$ are those known to be useful in the art in the step-wise synthesis of polypeptides. Among the classes of alpha-amino protecting groups covered by $X^1$ are (1) acyl-type protecting groups, such as formyl, acrylyl(Acr), benzoyl(Bz) and acetyl (Ac) which are preferably used only at the N-terminal; (2) aromatic urethan-type protecting groups, such as benzyloxycarbonyl(Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (BOC), diisopropylmethoxycarbonyl, isopropyloxycarbonyl, ethoxycarbonyl, allyloxycarbonyl; (4) cycloalkyl urethan-type protecting groups, such as fluorenylmethyloxycarbonyl(FMOC), cyclopentyloxycarbonyl, adamantyloxycarbonyl, and cyclohexyloxycarbonyl; and (5) thiourethan-type protecting groups, such as phenylthiocarbonyl. The preferred alpha-amino protecting group is BOC if the synthesis employs acid-catalyzed removal of the alpha-amino protecting groups; however, for syntheses employing a base-catalyzed removal strategy, FMOC is preferred, in which case more acid-labile side-chain protecting groups can be used, including t-Butyl esters or ethers as well as BOC.

$X^2$ is a protecting group for the hydroxyl group of Thr and Ser and is generally selected from the class containing acetyl(Ac), benzoyl(Bz), tert-butyl(t-Bu), triphenylmethyl(trityl), tetrahydropyranyl, benzyl ether(Bzl) and 2,6-dichlorobenzyl(DCB) when a BOC strategy is employed. The preferred protecting group is Bzl for a BOC strategy and t-Bu for FMOC strategy. $X^2$ can also be hydrogen, which means there is no protecting group on the hydroxyl group.

$X^3$ is a protecting group for the guanidino group of Arg generally selected from the class containing nitro, p-toluenesulfonyl (Tos), Z, adamantyloxycarbonyl and BOC, or is hydrogen. Tos is preferred for a BOC strategy and 4-methoxy-2,3,6-trimethyl benzene sulfonyl (MTR) or pentamethylchroman-6-sulfonyl(PMC) for FMOC strategy.

$X^4$ is hydrogen or a protecting group, preferably xanthyl(Xan), for the amido group of Asn or Gln.

$X^5$ is hydrogen or an ester-forming protecting group for the β- or γ-carboxyl group of Asp or Glu, and is generally selected from the class containing the esters of cyclohexyl(OChx), benzyl(OBzl), 2,6-dichlorobenzyl, methyl, ethyl and t-butyl(Ot-Bu). OChx is preferred for a BOC strategy and Ot-Bu for FMOC strategy.

$X^6$ is hydrogen or a protecting group for the side chain amino substituent of Lys. Illustrative of suitable side chain amino protecting groups are Z, 2-chlorobenzyloxycarbonyl(2-Cl-Z), Tos, t-amyloxycarbonyl(Aoc), BOC and aromatic or aliphatic urethan-type protecting groups as specified hereinbefore. 2-Cl-Z is preferred for a BOC strategy and BOC for FMOC strategy.

$X^7$ is hydrogen or a protecting group for the imidazole nitrogen of His such as Tos or 2,4-dinitrophenyl(DNP).

When Met is present, the sulfur may be protected, if desired, with oxygen.

The selection of a side chain amino protecting group is not critical except that it should must be one which is not removed during deprotection of the alpha-amino groups during the synthesis. Hence, the alpha-amino protecting group and the side chain amino protecting group cannot be the same.

$X^8$ is $NH_2$, a protecting group such as an ester or an anchoring bond used in solid phase synthesis for linking to a solid resin support, preferably one represented by the formulae:

-NH-benzhydrylamine (BHA) resin support and -NH-paramethylbenzhydrylamine (MBHA) resin support. Cleavage from a BHA or MBHA resin directly gives the CRF analog amide. By employing an N-methyl-derivative of such a resin, a methyl-substituted amide can be created.

In the formula for the intermediate, at least one of $X^1$, $X^2$, $X^3$, $X^4$, $X^5$, $X^6$ and $X^7$ is a protecting group. The particular amino acid chosen for each the R-group determines whether there will also be a protecting group attached as specified hereinbefore and as generally known in the art. In selecting a particular side chain protecting group to be used in the synthesis of the peptides, the following rules are followed: (a) the protecting group should be stable to the reagent and under the reaction conditions selected for removing the alpha-amino protecting group at each step of the synthesis, (b) the protecting group should retain its protecting properties and not be split off under coupling conditions and (c) the side chain protecting group must be removable, upon the completion of the synthesis containing the desired amino acid sequence, under reaction conditions that will not alter the peptide chain.

For the acyl group at the N-terminal represented by Y, acetyl, formyl, acrylyl and benzoyl are preferred. Moreover, as indicated hereinbefore, the N-terminus can be slightly shortened without significantly affecting biological potency.

Thus, there is also disclosed herein processes for the manufacture of compounds defined by the Formula (I)

4

comprising (a) forming a peptide intermediate having at least one protective group and having the Formula (IA) wherein: $X^1$, $X^2$, $X^3$, $X^4$, $X^5$, $X^6$ and $X^7$ are each either hydrogen or a protective group, and $X^8$ is either a protective group or an anchoring bond to resin support or $NH_2$ and (b) splitting off the protective group or groups or anchoring bond from said peptide intermediate of the Formula (II) and (c) if desired, converting a resulting peptide into a nontoxic addition salt thereof.

When the peptides are prepared by chemical synthesis, they are preferably prepared using solid phase synthesis, such as that described by Merrifield, J. Am. Chem. Soc., 85, p 2149 (1964), although other equivalent chemical syntheses known in the art can also be used as previously mentioned. Solid-phase synthesis is commenced from the C-terminus of the peptide by coupling a protected alpha-amino acid to a suitable resin as generally set forth in U.S. Patent No. 4,244,946 issued Jan. 21, 1981 to Rivier et al., the disclosure of which is incorporated herein by reference. Such a starting material for rCRF analogs can be prepared by attaching alpha-amino-protected Ile to a BHA resin.

Ile protected by BOC is coupled to the BHA resin using methylene chloride and dimethylformamide (DMF). Following the coupling of BOC-Ile to the resin support, the alpha-amino protecting group is removed, as by using trifluoroacetic acid(TFA) in methylene chloride, TFA alone or with HCl in dioxane. Preferably 50 volume % TFA in methylene chloride is used with 0-5 weight % 1,2 ethanedithiol. The deprotection is carried out at a temperature between about 0°C and room temperature. Other standard cleaving reagents and conditions for removal of specific alpha-amino protecting groups may be used as described in Schroder & Lubke, "The Peptides", 1 pp 72-75 (Academic Press 1965).

After removal of the alpha-amino protecting group of Ile, the remaining alpha-amino- and side chain-protected amino acids are coupled step-wise in the desired order to obtain the intermediate compound defined hereinbefore. As an alternative to adding each amino acid separately in the synthesis, some of them may be coupled to one another prior to addition to the solid phase reactor. The selection of an appropriate coupling reagent is within the skill of the art. Particularly suitable as coupling reagents are N,N'-dicyclohexyl carbodiimide(DCC) and N,N'-diisopropyl carbodiimide(DICI).

The activating reagents used in the solid phase synthesis of the peptides are well known in the peptide art. Examples of suitable activating reagents are carbodiimides, such as N,N'-diisopropyl carbodiimide and N-ethyl-N'-(3-dimethylaminopropyl) carbodiimide. Other activating reagents and their use in peptide coupling are described by Schroder & Lubke, supra, in Chapter III and by Kapoor, J. Phar. Sci., 59, pp 127 (1970).

Each protected amino acid or amino acid sequence is introduced into the solid phase reactor in about a fourfold excess, and the coupling is carried out in a medium of dimethylformamide(DMF):$CH_2Cl_2$ (1:1) or in DMF or $CH_2Cl_2$ alone. In instances where the coupling is carried out manually, the success of the coupling reaction at each stage of the synthesis is monitored by the ninhydrin reaction, as described by E. Kaiser et al., Anal. Biochem. 34, 595 (1970). In cases where incomplete coupling occurs, the coupling procedure is repeated before removal of the alpha-amino protecting group prior to the coupling of the next amino acid. The coupling reactions can be performed automatically, as on a Beckman 990 automatic synthesizer, using a program such as that reported in Rivier et al., Biopolymers, 1978, 17, pp.1927-1938.

After the desired amino acid sequence has been completed, the intermediate peptide is removed from the resin support by treatment with a reagent, such as liquid hydrogen fluoride, which not only cleaves the peptide from the resin but also cleaves all remaining side chain protecting groups $X^2$, $X^3$, $X^4$, $X^5$, $X^6$ and $X^7$ and the alpha-amino protecting group $X^1$ (unless it is an acyl group which is intended to be present in the final peptide) to obtain the peptide. When using hydrogen fluoride for cleaving, anisole or cresole and methylethyl sulfide are included in the reaction vessel as scavengers. When Met is present in the sequence, the BOC protecting group may be cleaved with trifluoroacetic acid (TFA)/ethanedithiol prior to cleaving the peptide from the resin to eliminate potential S-alkylation.

The following Example sets forth the preferred method for synthesizing CRF analogs by the solid-phase technique.

EXAMPLE I

The synthesis of the peptide below is conducted in a stepwise manner on a MBHA hydrochloride resin, such as available from Bachem, Inc., having a substitution range of about 0.1 to 0.5 mmoles/gm. Resin. The synthesis is performed on an automatic Beckman 990B peptide synthesizer using a suitable program, preferably as follows:-

| STEP | REAGENTS AND OPERATIONS) | MIX TIMES (MIN) |
|------|--------------------------|-----------------|
| 1 | $CH_2CL_2$ wash-80 ml. (2 times) | 3 |
| 2 | Methanol (MeOH) wash-30 ml. (2 times) | 3 |
| 3 | $CH_2CL_2$ wash-80 ml. (3 times) | 3 |
| 4 | 50 percent TFA plus 5 percent 1,2-ethane-dithiol in $CH_2CL_2$-70 ml. (2 times) | 12 |
| 5 | Isopropanol wash-80 ml. (2 times) | 3 |

(continued)

| STEP | REAGENTS AND OPERATIONS) | MIX TIMES (MIN) |
|---|---|---|
| 6 | TEA 12.5 percent in $CH_2CL_2$-70 ml. (2 times) | 5 |
| 7 | MeOH wash-40 ml. (2 times) | 2 |
| 8 | $CH_2CL_2$ wash-80 ml. (3 times) | 3 |
| 9 | Boc-amino acid (10 mmoles) in 30 ml. Of either DMF or $CH_2CL_2$, depending upon the solubility of the particular protected amino acid, (1 time) plus DCC (10 mmoles) in $CH_2CL_2$ | 30-300 |

Coupling of BOC-Ile results in the substitution o about 0.35 mmol. Ile per gram of resin. All solvents that are used are carefully degassed, preferably by sparging with an inert gas, e.g., helium or nitrogen, to insure the absence of oxygen that might undesirably oxidise the sulfur of the Met residue.

After deprotection and neutralization, the peptide chain is built step-by-step on the resin. Generally, one to two mmol. Of BOC-protected amino acid in methylene chloride is used per gram of resin, plus one equivalent of 2 molar DCC in methylene chloride, for two hours. When BOC-Arg(Tos) is being coupled, a mixture of 50% DMF and methylene chloride is used. Bzl is used as the hydroxyl side-chain protecting group for Ser and Thr. P-nitrophenyl ester(ONp) can be used to activate the carboxyl end of Asn or Gln; for example, BOC-Asn(ONp) can be coupled overnight using one equivalent of HOBt in a 50% mixture of DMF and methylene chloride. The amido group of Asn or Gln is protected by Xan when DCC coupling is used instead of the active ester method. 2-Cl-Z is used as the protecting group for the Lys side chain. Tos is used to protect the guanidino group of Arg and the imidazole group of His, and the side-chain carboxyl group of Glu or Asp is protected by OBzl. At the end of the synthesis, the following composition is obtained: BOC-D-Pro-Pro-Ile-Ser(Bzl)-Leu-Asp(OBzl)-Leu-Thr(Bzl)D-Phe-His(Tos)-Leu-Leu-Arg(Tos)-Glu(OBzl)-Val-Leu-Glu(OBzl)-Nle-Ala-Arg(Tos)-Ala-Glu(OBzl)-Gln(Xan)-Leu-Ala-Gln(Xan)-Gln(Xan)-Ala-His(Tos)-Ser(Bzl)-Asn(Xan)-Arg(Tos)-Lys(2-Cl-Z)-CML-Nle-Glu(OBzl)-Ile-Ile-resin support. Xan may have been partially or totally removed by TFA treatment used to deblock the alpha-amino protecting group.

In order to cleave and deprotect the resulting protected peptide-resin, it is treated with 1.5 ml. Anisole, 0.5 ml. of methylethylsulfide and 15 ml. Hydrogen fluoride (HF) per gram of peptide-resin, first at -20°C for 20 min. And then at 0°C for one-half hour. After elimination of the HF under high vacuum, the resin-peptide is washed alternately with dry diethyl ether and chloroform, and the peptides are then extracted with degassed 2N aqueous acetic acid and separated from the resin by filtration.

The peptide is purified by gel permeation followed by preparative HPLC as described in Marki, et al., J. Am. Chem. Soc., 103, 3178 (1981); Rivier, et al., J. Chromatography, 288, 303-328 (1984); and Hoeger, et al., BioChromatography, 2, 3, 134-142 (1987). The chromatographic fractions are carefully monitored by HPLC, and only the fractions showing substantial purity are pooled.

To check whether the precise sequence is achieved, the rCRF analog is hydrolyzed in sealed evacuated tubes containing constant boiling HCl, 3μl of thioglycol/ml. And 1 nmol of Nle (as an internal standard) for 9 hours at 140ºC. Amino acid analysis of the hydrolysates using a Beckman 121 MB amino acid analyzer shows amino acid ratios which confirm that the 41-residue peptide structure has been obtained. The peptide [D-Pro[4], D-Phe[12], Nle[21, 38], CML[37]] - rCRF (4-41) having the formula: H-D-Pro-Pro-Ile-Ser-Leu-Asp-Leu-Thr-D-Phe-His-Leu-Leu-Arg-Glu-Val-Leu-Glu-Nle-Ala-Arg-Ala-Glu-Gln-Leu-Ala-Gln-Gln-Ala-His-Ser-Asn-Arg-Lys-CML-Nle-Glu-Ile-Ile-$NH_2$ is synthesized.

The peptide is judged to be homogeneous following being specifically subjected to reversed-phase high performance liquid chromatography using a Waters HPLC system with a 0.45 x 25 cm. column packed with 5μm $C_{18}$ silica, 300A pore size. Buffer A which is used is an aqueous triethylammonium phosphate (TEAP) solution having a pH of 3.0, containing 5% $CH_3CN$; Buffer B is a mixture of 20% pH 3.0 TEAP and 80% acetonitrile. The determination is run at room temperature with a gradient from 30% Buffer B to 60% Buffer B over 30 minutes. The flow rate is 2.0 ml. per minute, and the retention time is 20.79 minutes.

Specific optical rotation of the rCRF peptide, which was synthesized and purified in the foregoing manner, is measured on a Perkin Elmer Model 241 Polarimeter as $[\alpha]_D^{22}$ = -32.9 ± 1.0 (c=1 in 1% acetic acid). It had a purity of greater than 98%.

Amino acid analysis of the resultant, purified peptide is consistent with the formula for the prepared peptide and confirms that the 37-residue peptide structure is obtained.

This synthetic peptide is examined for its effects on the secretion of ACTH and β-endorphin in vitro and also in vivo. The potency of the synthetic peptide to stimulate the secretion of ACTH and β-endorphin by cultured rat pituitary cells is measured using the procedure as generally set forth in Endocrinology, 91, 562 (1972) and compared against synthetic oCRF. Half-maximal responses are observed at about 170 picomolar concentrations of this peptide, while synthetic oCRF concentrations of about 250 picomolar are needed to achieve this response. The secretory response to maximal (1-5nM) concentrations of this peptide plateau at a level about twice that of the native hormone. In vivo testing is carried out using the general procedure set forth in C. Rivier et al., Science, 218, 377 (1982) and shows a very substantially longer duration of potency than the CRF standard.

EXAMPLE II

The peptide [Acetyl-Ser$^1$, D-Phe$^{12}$, Nle$^{21,38}$, CML$^{37}$]-rCRF having the formula:
Ac-Ser-Glu-Glu-Pro-Pro-Ile-Ser-Leu-Asp-Leu-Thr-D-Phe-His-Leu-Leu-Arg-Glu-Val-Leu-Glu-Nle-Ala-Arg-Ala-Glu-Gln-Leu-Ala-Gln-Gln-Ala-His-Ser-Asn-Arg-Lys-CML-Nle-Glu-Ile-Ile-NH$_2$ is synthesized. Amino acid analysis of the resultant, purified peptide is consistent with the formula for the prepared peptide and confirms that the 41-residue peptide structure is obtained. Testing in accordance with the general procedure set forth hereinbefore shows that it likewise stimulates the secretion of ACTH and β-END-LI and causes a very significant lowering of blood pressure.

EXAMPLE III

The peptide [Benzoyl-Ser$^7$, D-Phe$^{12}$, Nle$^{21,38}$, D-His$^{32}$ CML$^{37}$]-rCRF having the formula:
Bz-Ser-Leu-Asp-Leu-Thr-D-Phe-His-Leu-Leu-Arg-Glu-Val-Leu-Glu-Nle-Ala-Arg-Ala-Glu-Gln-Leu-Ala-Gln-Gln-Ala-D-His-Ser-Asn-Arg-Lys-CML-Nle-Glu-Ile-Ile-NH$_2$ is synthesized. Amino acid analysis of the resultant, purified peptide is consistent with the formula for the prepared peptide and confirms that the 41-residue peptide structure is obtained. Testing in accordance with the general procedure set forth hereinbefore shows that it likewise stimulates the secretion of ACTH and β-END-LI and causes a very significant lowering of blood pressure.

EXAMPLE IV

The peptide [Acrylyl-Glu$^2$, D-Phe$^{12}$, Nle$^{21,38}$, D-His$^{32}$, CML$^{37}$]-rCRF(2-41) having the formula:
Acr-Glu-Glu-Pro-Pro-Ile-Ser-Leu-Asp-Leu-Thr-D-Phe-His-Leu-Leu-Arg-Glu-Val-Leu-Glu-Nle-Ala-Arg-Ala-Glu-Gln-Leu-Ala-Gln-Gln-Ala-D-His-Ser-Asn-Arg-Lys-CML-Nle-Glu-Ile-Ile-NH$^2$ is synthesized. Amino acid analysis of the resultant, purified peptide is consistent with the formula for the prepared peptide and confirms that the 40-residue peptide structure is obtained. Testing in accordance with the general procedure set forth hereinbefore shows that it likewise stimulates the secretion of ACTH and β-END-LI and causes a very significant lowering of blood pressure.

EXAMPLE V

The peptide [D-Phe$^{12}$, Nle$^{21,38}$, CML$^{37}$]-oCRF having the formula:
H-Ser-Gln-Glu-Pro-Pro-Ile-Ser-Leu-Asp-Leu-Thr-D-Phe-His-Leu-Leu-Arg-Glu-Val-Leu-Glu-Nle-Thr-Lys-Ala-Asp-Gln-Leu-Ala-Gln-Gln-Ala-His-Ser-Asn-Arg-Lys-CML-Nle-Asp-Ile-Ala-NH$_2$ is synthesized using a procedure generally as set forth in Example I. Amino acid analysis of the resultant, purified peptide is consistent with the formula for the prepared peptide and confirms that the 41-residue peptide structure is obtained. Testing in accordance with the general procedure set forth hereinbefore shows that it likewise stimulates the secretion of ACTH and β-END-LI and causes a very significant lowering of blood pressure.

CRF profoundly stimulates the pituitary-adrenalcortical axis, and CRF analogs should be useful to stimulate the functions of this axis in some types of patients with low endogenous glucocorticoid production. For example, CRF should be useful in restoring pituitary-adrenal function in patients having received exogenous glucocorticoid therapy whose pituitary-adrenalcortical functions remain suppressed.

Most other regulatory peptides have been found to have effects upon the central nervous system and upon the gastrointestinal tract. Because ACTH and β-END secretion is the "sine qua non" of mammal's response to stress, it was not surprising that CRF has significant effects on the brain as a mediator of the body's stress response. For example, CRF in the brain appears to increase respiratory rate and may be useful in treating respiratory depression. CRF may also find application in modifying the mood, learning and behavior of normal and mentally disordered individuals. Because CRF analogs elevate the levels of ACTH, β-END, β-lipotropin, other pro-opiomelanocortin gene products and corticosterone, its administration can be used to induce their effects on the brain and periphery to thereby influence memory, mood, pain appreciation, etc., and more specifically, alertness, depression and/or anxiety. For example, when administered into the ventricles, CRF increases activity and improves learning performance in rats and thus may function as a natural stimulant.

CRF analogs should also be of use for increasing blood flow to the gastrointestinal tract of mammals, particularly humans and other mammals. All CRF related peptides have been shown to dialate the mesenteric vascular bed. Also, oCRF inhibits gastric acid production, and CRF analogs are expected to also be effective in the treatment of gastric ulcers by reducing gastric acid production and/or inhibiting gastrointestinal functions in a mammal.

CRF analogs or the nontoxic addition salts thereof, combined with a pharmaceutically or veterinarily acceptable carrier to form a pharmaceutical composition, may be administered to mammals, including humans, either intravenously, subcutaneously, intramuscularly, percutaneously, e.g. intranasally, intracerebrospinally or orally. The peptides should be at least about 90% pure and preferably should have a purity of at least about 98%; however, lower purities are effective and may well be used with mammals other than humans. This purity means that the intended peptide constitutes the stated weight percent of all like peptides and peptide fragments present. Administration to humans may be employed by a physician to lower blood pressure or to stimulate endogenous gluco-corticoid production. The required dosage will vary with the particular condition being treated, with the severity of the condition and with the duration of desired treatment.

These peptides may also be used to evaluate hypothalamic pituitary adrenal function in mammals with suspected endocrine or central nervous system pathology by suitable administration followed by monitoring body functions. For example, administration may be used as a diagnostic tool to evaluate Cushing's disease and affective disorders, such as depressive illness.

Such peptides are often administered in the form of pharmaceutically or veterinarily acceptable nontoxic salts, such as acid addition salts or metal complexes, e.g., with zinc, iron, calcium, barium, magnesium, aluminum or the like (which are considered as addition salts for purposes of this application). Illustrative of such acid addition salts are hydrochloride, hydrobromide, sulphate, phosphate, tannate, oxalate, fumarate, gluconate, alginate, maleate, acetate, citrate, benzoate, succinate, malate, ascorbate, tartrate and the like. If the active ingredient is to be administered in tablet form, the tablet may contain a binder, such as tragacanth, corn starch or gelatin; a disintegrating agent, such as alginic acid; and a lubricant, such as magnesium stearate. If administration in liquid form is desired, sweetening and/ or flavoring may be used, and intravenous administration in isotonic saline, phosphate buffer solutions or the like may be effected.

The peptides should be administered under the guidance of a physician, and pharmaceutical compositions will usually contain the peptide in conjunction with a conventional, pharmaceutically or veterinarily-acceptable carrier. Usually, the dosage will be from about 1 to about 200 micrograms of the peptide per kilogram of the body weight of the host animal. In some instances, treatment of subjects with these peptides can be carried out in lieu of the administration of ACTH or corticosteroids, in such instances a dosage as low as about 10 ng/Kg of body weight may be employed. As used herein, all temperatures are °C and all ratios are by volume. Percentages of liquid materials are also by volume.

## Claims

### Claims for the following Contracting States : AT, BE, CH, DE, DK, FR, GB, IT, LI, LU, NL, SE

1. A peptide having the formula:
Z-D-Pro-Pro-Ile-Ser-Leu-Asp-Leu-Thr-D-Phe-His-Leu-Leu-Arg-Glu-Val-Leu-$R_{20}$-Nle-$R_{22}$-$R_{23}$-$R_{24}$-$R_{25}$-Gln-Leu-Ala-Gln-Gln-Ala-$R_{32}$-Ser-Asn-Arg-Lys-CML-Nle-$R_{39}$-Ile-$R_{41}$-Y wherein $R_{20}$ is Glu or D-Glu; $R_{22}$ is Ala or Thr; $R_{23}$ is Arg or Lys; $R_{24}$ is Ala or D-Ala; $R_{25}$ is Glu or Asp; $R_{32}$ is His or D-His; $R_{39}$ is Glu or Asp; $R_{41}$ is Ile or Ala; Y is amino, methylamino or ethylamino; Z is an acyl group of up to seven carbon atoms or is hydrogen; or a nontoxic addition salt thereof.

2. The peptide of Claim 1 wherein $R_{22}$ is Ala, $R_{23}$ is Arg, $R_{25}$ is Glu, $R_{39}$ is Glu and $R_{41}$ is Ile.

3. The peptide of Claim 1 or 2 wherein $R_{20}$ is D-Glu.

4. The peptide of Claim 1 wherein $R_{22}$ is Thr, $R_{23}$ is Lys, $R_{25}$ is Asp, $R_{39}$ is Asp and $R_{41}$ is Ala.

5. The peptide of Claim 1 having the formula:
H-D-Pro-Pro-Ile-Ser-Leu-Asp-Leu-Thr-D-Phe-His-Leu-Leu-Arg-Glu-Val-Leu-Glu-Nle-Ala-Arg-Ala-Glu-Gln-Leu-Ala-Gln-Gln-Ala-His-Ser-Asn-Arg-Lys-CML-Nle-Glu-Ile-Ile-$NH_2$.

### Claims for the following Contracting States : ES, GR

1. A method for making a peptide or a nontoxic salt thereof, said peptide having the formula: Z-D-Pro-Pro-Ile-Ser-Leu-Asp-Leu-Thr-D-Phe-His-Leu-Leu-Arg-Glu-Val-Leu-$R_{20}$-Nle-$R_{22}$-$R_{23}$-$R_{24}$-$R_{25}$-Gln-Leu-Ala-Gln-Gln-Ala-$R_{32}$-Ser-Asn-Arg-Lys-CML-Nle-$R_{39}$-Ile-$R_{41}$-Y wherein $R_{20}$ is Glu or D-Glu; $R_{22}$ is Ala or Thr; $R_{23}$ is Arg or Lys; $R_{24}$ is

Ala or D-Ala; $R_{25}$ is Glu or Asp; $R_{32}$ is His or D-His; $R_{39}$ is Glu or Asp; $R_{41}$ is Ile or Ala; Y is amino, methylamino or ethylamino; Z is an acyl group of up to seven carbon atoms or is hydrogen; comprising (a) forming a peptide intermediate having at least one protective group and having the formula: $X^1$-D-Pro-Pro-Ile-Ser($X^2$)-Leu-Asp($X^5$)-Leu-Thr($X^2$)-D-Phe-His($X^7$)-Leu-Leu-Arg($X^3$)-Glu($X^5$)-Val-Leu-$R_{20}$($X^5$)-Nle-$R_{22}$($X^2$)-$R_{23}$($X^3$ or $X^6$)-$R_{24}$-$R_{25}$($X^5$)-Gln($X^4$)-Leu-Ala-Gln($X^4$)-Gln($X^4$)-Ala-$R_{32}$($X^7$)-Ser($X^2$)-Asn($X^4$)-Arg($X^3$)-Lys($X^6$)-CML-Nle-$R_{39}$($X^5$)-Ile-$R_{41}$-($X^8$) or an N-terminally shortened version thereof, wherein $X^1$, $X^2$, $X^3$, $X^4$, $X^5$, $X^6$, and $X^7$ are each either hydrogen or a protective group, and $X^8$ is either a protective group or an anchoring bond to resin support or $NH_2$ and (b) splitting off the protective group or groups or anchoring bond from said peptide intermediate and (c) if desired, converting a resulting peptide into a nontoxic salt thereof.

2. The method of Claim 1 wherein $R_{22}$ is Ala, $R_{23}$ is Arg, $R_{25}$ is Glu, $R_{39}$ is Glu and $R_{41}$ is Ile.

3. The method of Claim 1 or 2 wherein $R_{20}$ is D-Glu.

4. The method of Claim 1 wherein $R_{22}$ is Thr, $R_{23}$ is Lys, $R_{25}$ is Asp, $R_{39}$ is Asp and $R_{41}$ is Ala.


**Patentansprüche**


**Patentansprüche für folgende Vertragsstaaten : AT, BE, CH, DE, DK, FR, GB, IT, LI, LU, NL, SE:**

1. Peptid der Formel:
   Z-D-Pro-Pro-Ile-Ser-Leu-Asp-Leu-Thr-D-Phe-His-Leu-Leu-Arg-Glu-Val-Leu-$R_{20}$-Nle-$R_{22}$-$R_{23}$-$R_{24}$-$R_{25}$-Gln-Leu-Ala-Gln-Gln-Ala-$R_{32}$-Ser-Asn-Arg-Lys-CML-Nle-$R_{39}$-Ile-$R_{41}$-Y, worin $R_{20}$ Glu oder D-Glu ist; $R_{22}$ Ala oder Thr ist; $R_{23}$ Arg oder Lys ist; $R_{24}$ Ala oder D-Ala ist; $R_{25}$ Glu oder Asp ist; $R_{32}$ His oder D-His ist; $R_{39}$ Glu oder Asp ist; $R_{41}$ Ile oder Ala ist; Y Amino, Methylamino oder Ethylamino ist; Z eine Acylgruppe mit bis zu sieben Kohlenstoffatomen oder Wasserstoff ist; oder nichttoxisches Salz davon.

2. Peptid nach Anspruch 1, worin $R_{22}$ Ala ist, $R_{23}$ Arg ist, $R_{25}$ Glu ist, $R_{39}$ Glu ist und $R_{41}$ Ile ist.

3. Peptid nach Anspruch 1 oder 2, worin $R_{20}$ D-Glu ist.

4. Peptid nach Anspruch 1, worin $R_{22}$ Thr ist, $R_{23}$ Lys ist, $R_{25}$ Asp ist, $R_{39}$ Asp ist und $R_{41}$ Ala ist.

5. Peptid nach Anspruch 1 der Formel:
   H-D-Pro-Pro-Ile-Ser-Leu-Asp-Leu-Thr-D-Phe-His-Leu-Leu-Arg-Glu-Val-Leu-Glu-Nle-Ala-Arg-Ala-Glu-Gln-Leu-Ala-Gln-GlnAla-His-Ser-Asn-Arg-Lys-CML-Nle-Glu-Ile-Ile-$NH_2$.


**Patentansprüche für folgende Vertragsstaaten : ES, GR**

1. Verfahren zur Herstellung eines Peptids oder nichttoxischen Salzes davon, welches Peptid die Formel: Z-D-Pro-Pro-Ile-Ser-Leu-Asp-Leu-Thr-D-Phe-His-Leu-Leu-Arg-Glu-Val-Leu-$R_{20}$-Nle, $R_{22}$-$R_{23}$-$R_{24}$-$R_{25}$-Gln-Leu-Ala-Gln-Gln-Ala-$R_{32}$-Ser-Asn-Arg-Lys-CML-Nle-$R_{39}$-Ile-$R_{41}$-Y hat, worin $R_{20}$ Glu oder D-Glu ist; $R_{22}$ Ala oder Thr ist; $R_{23}$ Arg oder Lys ist; $R_{24}$ Ala oder D-Ala ist; $R_{25}$ Glu oder Asp ist; $R_{32}$ His oder D-His ist; $R_{39}$ Glu oder Asp ist; $R_{41}$ Ile oder Ala ist; Y Amino, Methylamino oder Ethylamino ist; Z eine Acylgruppe mit bis zu sieben Kohlenstoffatomen oder Wasserstoff ist; umfassend (a) das Bilden einer Peptid-Intermediärsubstanz mit mindestens einer Schutzgruppe und der Formel: $X^1$-D-Pro-Pro-Ile-Ser($X^2$)-Leu-Asp($X^5$)-Leu-Thr($X^2$)-D-Phe-His($X^7$)-Leu-Leu-Arg($X^3$)-Glu($X^5$)-Val-Leu-$R_{20}$($X^5$)-Nle-$R_{22}$($X^2$)-$R_{23}$($X^3$ oder $X^6$)-$R_{24}$-$R_{25}$($X^5$)-Gln($X^4$)-Leu-Ala-Gln($X^4$)-Gln($X^4$)-Ala-$R_{32}$ ($X^7$)-Ser($X^2$)-Asn($X^4$)-Arg($X^3$)-Lys($X^6$)-CML-Nle-$R_{39}$ ($X^5$)-Ile-$R_{41}$-($X^8$) oder einer N-terminal verkürzte Version davon, worin $X^1$, $X^2$, $X^3$, $X^4$, $X^5$, $X^6$ und $X^7$ jeweils entweder Wasserstoff oder eine Schutzgruppe sind und $X^8$ entweder eine Schutzgruppe oder eine Ankerbindung an einen Harzträger oder $NH_2$ ist, und (b) das Abspalten der Schutzgruppe(n) oder Ankerbindung von der Peptid-Intermediärsubstanz und (c) gewünschtenfalls das Überführen eines resultierenden Peptids in ein nichttoxisches Salz davon.

2. Verfahren nach Anspruch 1, worin $R_{22}$ Ala ist, $R_{23}$ Arg ist, $R_{25}$ Glu ist, $R_{39}$ Glu ist und $R_{41}$ Ile ist.

**3.** Verfahren nach Anspruch 1 oder 2, worin $R_{20}$ D-Glu ist.

**4.** Verfahren nach Anspruch 1, worin $R_{22}$ Thr ist, $R_{23}$ Lys ist, $R_{25}$ Asp ist, $R_{39}$ Asp ist und $R_{41}$ Ala ist.

## Revendications

**Revendications pour les Etats contractants suivants : AT, BE, CH, DE, DK, FR, GB, IT, LI, LU, NL, SE**

**1.** Peptide ayant la formule :
Z - D - Pro - Pro - Ile - Ser - Leu - Asp - Leu - Thr - D - Phe - His - Leu - Leu - Arg - Glu - Val - Leu - $R_{20}$ - Nle - $R_{22}$ - $R_{23}$ - $R_{24}$ - $R_{25}$ - Gln - Leu - Ala - Gln - Gln - Ala - $R_{32}$ - Ser - Asn - Arg - Lys - CML - Nle - $R_{39}$ - Ile - $R_{41}$ - Y où $R_{20}$ est Glu ou D - Glu ; $R_{22}$ est Ala ou Thr ; $R_{23}$ est Arg ou Lys ; $R_{24}$ est Ala ou D - Ala ; $R_{25}$ est Glu ou Asp ; $R_{32}$ est His ou D - His ; $R_{39}$ est Glu ou Asp ; $R_{41}$ est Ile ou Ala ; Y est un groupe amino, méthylamino ou éthylamino ; Z est un groupe acyle ayant jusqu'à sept atomes de carbone ou est l'hydrogène ; ou un sel d'addition non toxique de celui - ci.

**2.** Peptide selon la revendication 1 dans lequel $R_{22}$ est Ala, $R_{23}$ est Arg, $R_{25}$ est Glu, $R_{39}$ est Glu et $R_{41}$ est Ile.

**3.** Peptide selon la revendication 1 ou 2 dans lequel $R_{20}$ est D - Glu.

**4.** Peptide selon la revendication 1 dans lequel $R_{22}$ est Thr, $R_{23}$ est Lys, $R_{25}$ est Asp, $R_{39}$ est Asp et $R_{41}$ est Ala.

**5.** Peptide selon la revendication 1 ayant la formule :
H - D - Pro - Pro - Ile - Ser - Leu - Asp - Leu - Thr - D - Phe - His - Leu - Leu - Arg - Glu - Val - Leu - Glu - Nle - Ala - Arg - Ala - Glu - Gln - Leu - Ala - Gln - Gln - Ala - His - Ser - Asn - Arg - Lys - CML - Nle - Glu - Ile - Ile - $NH_2$.

**Revendications pour les Etats contractants suivants : ES, GR**

**1.** Procédé de fabrication d'un peptide ou d'un sel non toxique de celui-ci, ledit peptide ayant la formule : Z - D - Pro - Pro - Ile - Ser - Leu - Asp - Leu - Thr - D - Phe - His - Leu - Leu - Arg - Glu - Val - Leu - $R_{20}$ - Nle - $R_{22}$ - $R_{23}$ - $R_{24}$ - $R_{25}$ - Gln - Leu - Ala - Gln - Gln - Ala - $R_{32}$ - Ser - Asn - Arg - Lys - CML - Nle - $R_{39}$ - Ile - $R_{41}$ - Y où $R_{20}$ est Glu ou D - Glu ; $R_{22}$ est Ala ou Thr ; $R_{23}$ est Arg ou Lys ; $R_{24}$ est Ala ou D - Ala ; $R_{25}$ est Glu ou Asp ; $R_{32}$ est His ou D - His ; $R_{39}$ est Glu ou Asp ; $R_{41}$ est Ile ou Ala ; Y est un groupe amino, méthylamino ou éthylamino ; Z est un groupe acyle pouvant avoir jusqu'à sept atomes de carbone ou est l'hydrogène ; comprenant (a) l'étape consistant à former un intermédiaire peptidique ayant au moins un groupe de protection et ayant la formule : $X^1$ - D - Pro - Pro - Ile - Ser ($X^2$) - Leu - Asp ($X^5$) - Leu - Thr ($X^2$) - D - Phe - His ($X^7$) - Leu - Leu - Arg ($X^3$) - Glu ($X^5$) - Val - Leu - $R_{20}$ ($X^5$) - Nle - $R_{22}$ ($X^2$) - $R_{23}$ ($X^3$ ou $X^6$) - $R_{24}$ - $R_{25}$ ($X^5$) - Gln ($X^4$) - Leu - Ala - Gln ($X^4$) - Gln ($X^4$) - Ala - $R_{32}$ ($X^7$) - Ser ($X^2$) - Asn ($X^4$) - Arg ($X^3$) - Lys ($X^6$) - CML - Nle - $R_{39}$ ($X^5$) - Ile - $R_{41}$ - ($X^8$) ou une version réduite de celle-ci en région N - terminale, où chacun des $X^1$, $X^2$, $X^3$, $X^4$, $X^5$, $X^6$ et $X^7$ sont l'hydrogène ou bien un groupe de protection et $X^8$ est un groupe de protection ou une liaison d'ancrage à un support résinique ou $NH_2$ et (b) la scission du groupe ou des groupes de protection ou de la liaison d'ancrage dudit peptide intermédiaire et (c), si on le souhaite, la conversion d'un peptide résultant en un sel non toxique de celui-ci.

**2.** Procédé selon la revendication 1 dans lequel $R_{22}$ est Ala, $R_{23}$ est Arg, $R_{25}$ est Glu, $R_{39}$ est Glu et $R_{41}$ est Ile.

**3.** Procédé selon la revendication 1 ou 2 dans lequel $R_{20}$ est D - Glu.

**4.** Procédé selon la revendication 1 dans lequel $R_{22}$ est Thr, $R_{23}$ est Lys, $R_{25}$ est Asp, $R_{39}$ est Asp et $R_{41}$ est Ala.